# EUROPÄISCHE PATENTSCHRIFT

(11) **EP 3 870 107 B1**
(45) Veröffentlichungstag und Bekanntmachung des Hinweises auf die Patenterteilung: **14.06.2023**
(21) Anmeldenummer: 19779774.9
(22) Anmeldetag: 20.09.2019
(51) Int. Cl.: A61F 2/16, A61K 31/00, C07K 16/22

(54) **OPHTHALMOLOGISCHES IMPLANTAT, VERFAHREN ZUM HERSTELLEN EINES OPHTHALMOLOGISCHEN IMPLANTATS UND VERWENDUNG EINES LIGANDEN ZUR HERSTELLUNG EINES OPHTHALMOLOGISCHEN IMPLANTATS**
OPHTHALMOLOGIC IMPLANT, METHOD OF MAKING AN OPHTHALMOLOGIC IMPLANT AND USE OF A LIGAND FOR MAKING AN OPHTHALMOLOGIC IMPLANT
IMPLANT OPHTALMOLOGIQUE, PROCÉDÉ DE FABRICATION D'UN IMPLANT OPHTALMOLOGIQUE ET UTILISATION D'UN LIGAND POUR FABRIQUER UN IMPLANT OPHTALMOLOGIQUE

(30) Priorität: 26.10.2018 DE 102018126842
(43) Veröffentlichungstag der Anmeldung: 01.09.2021
(73) Patentinhaber: Carl Zeiss Meditec AG, 07745 Jena (DE)
(72) Erfinder: WOLFSTEIN, André, 13507 Berlin (DE)
(74) Vertreter: Hofstetter, Schurack & Partner
(86) Internationale Anmeldenummer: PCT/EP2019/075325
(87) Internationale Veröffentlichungsnummer: WO 2020/083582

(56) Entgegenhaltungen:
- EP-A1- 3 202 369
- WO-A1-2013/040047
- DE-A1-102008 017 592
- US-A1- 2004 047 900
- US-A1- 2012 015 001
- US-A1- 2018 057 578
- Huang Yi-Shiang ET AL: "RGD Surface Functionalization of the Hydrophilic Acrylic Intraocular Lens Material to Control Posterior Capsular Opacification", PLoS ONE, vol. 9, no. 12, 11 December 2014 (2014-12-11), pages 1-32, XP055904629, DOI: 10.1371/journal.pone.0114973

## Beschreibung

### Technisches Gebiet

Die Erfindung betrifft ein ophthalmologisches Implantat, ein Verfahren zum Herstellen eines ophthalmologischen Implantats sowie eine Verwendung eines Liganden zur Herstellung eines ophthalmologischen Implantats.

### Stand der Technik

Als Fibrose (fachsprachlich auch Fibrosis) wird eine krankhafte Vermehrung des Bindegewebes in menschlichen und tierischen Geweben bezeichnet, dessen Hauptbestandteil Kollagenfasern sind. Dabei wird das Gewebe des betroffenen Organs verhärtet. Es entstehen narbige Veränderungen, die im fortgeschrittenen Stadium zur Einschränkung der jeweiligen Organfunktion führen. Bei der Verwendung von ophthalmologischen Implantaten, beispielsweise von Intraokularlinsen können aufgrund von Wechselwirkungen zwischen dem Implantat und angrenzendem biologischem Gewebe verschiedene Komplikationen auftreten, von denen insbesondere solche Fibrosen problematisch sind. Beispielsweise tritt nach Katarakt-Operationen in manchen Fällen eine sogenannte posteriore Kapselopazifikation (posterior capsule opacification, PCO, Cataracta secundaria) auf. Bei PCO handelt es sich um eine postoperative Trübung der Linsenkapsel nach der chirurgischen Extraktion einer natürlichen Linse. Die verbleibenden Linsenepithelzellen (E-Zellen) in der äquatorialen Region des Kapselsacks sind mitotisch aktiv und können zu Fibroblasten transformieren. Diese lösen dann eine Art Wundheilung aus, wobei kollagenhaltiges Bindegewebe gebildet wird.

Da manche Fibroplasten-Subtypen nicht nur an die Innenseite des Kapselsacks migrieren, sondern sich auch zusammenziehen können, kommt es zur Faltenbildung im Kapselsack. Die Trübung der Kapsel ist somit die Folge eines Wundheilungsprozesses und einer damit verbundenen Narbenbildung. Da die dadurch verursachte Linsentrübung andere Ursachen als die ursprüngliche Katarakt-Erkrankung besitzt, spricht man von einem "Nachstar" oder einem "sekundären Katarakt". Ein klinisch signifikanter Nachstar kann bei den Betroffenen zu einer Minderung der Sehschärfe, der Farbwahrnehmung und des Kontrastsehens sowie zu einer erhöhten Blendung führen.

Beim sekundären Katarakt handelt es sich um eine häufige Komplikation nach extrakapsulären Kataraktoperation (ECCE) und der anschließenden Implantation einer Intraokularlinse (IOL) in den Kapselsack. Ohne die Implantation einer IOL in den leeren Kapselsack ist das Risiko eines Nachstars sogar noch erheblich erhöht, da in diesem Fall eine ungehinderte Zellmigration zur hinteren Oberfläche des Kapselsackes möglich ist.

Die Inzidenz eines sekundären Katarakts erhöht sich nach einem operativen Eingriff mit der Zeit. Eine Meta-Analyse der Fälle von Cataracta secundaria für alle bestehenden Arten von IOLs zeigte postoperativ eine ungefähre durchschnittliche Zunahme von 12% nach einem Jahr und eine ungefähre durchschnittliche Zunahme von 30% nach fünf Jahren. Ein entscheidender Faktor scheint hierbei auch das Alter des betreffenden Patienten zu sein, so dass damit gerechnet werden muss, dass fast alle behandelten Kinder und Jugendlichen nach einer gewissen Zeit unter postoperativem sekundären Katarakt leiden könnten.

Die EP 3 202 369 A1 offenbart eine Intraokularlinse mit einem haptischen und einem optischen Teil. Die Intraokularlinse umfasst Quantenpunkte, die als Bestandteil einer Polymerbeschichtung mit Liganden versehen werden können.

Die US 2004/047900 A1 offenbart eine Behandlung der Trübung der hinteren Linse mittels Verwendung einer Polymerbeschichtung für Intraokularlinsen. Der Ligand ist dabei kovalent an das Polymer gebunden und kann ein Fas-Ligand oder ein Fas-Antikörper sein.

Die WO 2013/040047 A1 offenbart offenbart einen auf Seide basierenden Film, der als Beschichtung auf eine Intraokularlinse aufgetragen werden kann. Auf dem Film kann eine biologisch aktive Substanz angebracht werden.

Die US 2018/057578 A1 offenbart die Verwendung von TGF-β1-, TGF-β2- und/oder T TGF-β3-Antikörpern bei der Behandlung von Fibrose im Auge. Die Antikörper können kovalent an ein Biopolymer gebunden werden.

Die US 2012/015001 A1 offenbart offenbart medizinisch wirksame Substanzen, wie anti-TGF-β-Antikörper, die mittels Polymermatrix in das Auge implantierbar sind und antifibrotisch wirken.

Es ist bisher nicht möglich, im Rahmen von Augenoperationen alle Epithelzellen quantitativ zu entfernen, ohne toxische Arzneimittel wie beispielsweise 5-Fluoruracil, Thapsigargin oder Paclitaxel zu verwenden, die die Endothelzellschicht der Hornhaut oder anderes Augengewebe schädigen können. Die durch Fibrose und PCO hervorgerufenen Sehprobleme werden teilweise durch eine sogenannte fokale Nd:YAG-Kapsulotomie behandelt. Trotzdem könnten insbesondere fibrotische Striae weiterhin die Funktion akkommodationsfähiger IOLs beeinträchtigen.

### Darstellung der Erfindung

Aufgabe der vorliegenden Erfindung ist es, ein ophthalmologisches Implantat zu schaffen, welches das Risiko von PCO und Fibrose verringert. Eine weitere Aufgabe der Erfindung ist es, die Herstellung eines solchen ophthalmologischen Implantats zu ermöglichen.

Die Aufgaben werden erfindungsgemäß durch ein ophthalmologisches Implantat gemäß Patentanspruch 1, durch ein Verfahren zum Herstellen eines ophthalmologischen Implantats gemäß Patentanspruch 9 sowie durch eine Verwendung gemäß Patentanspruch 12 gelöst. Vorteilhafte Ausgestaltungen mit zweckmäßigen Ausbildungen der Erfindung sind in den jeweiligen Unteransprüchen angegeben, wobei vorteilhafte Ausgestaltungen jedes Erfindungsaspekts als vorteilhafte Ausgestaltungen der jeweils anderen Erfindungsaspekte anzusehen sind.

Ein erster Aspekt der Erfindung betrifft ein ophthalmologisches Implantat gemäß Anspruch 1 mit einem Grundkörper und wenigstens einem am Grundkörper immobilisierten Ligand, mittels welchem im implantierten Zustand des ophthalmologischen Implantats zumindest ein Fibrogen und/oder Zytokin zu binden und/oder zu deaktivieren ist. Mit anderen Worten ist es vorgesehen, dass das erfindungsgemäße ophthalmologische Implantat bzw. sein Grundkörper wenigstens einen bezüglich des Grundkörpers immobilisierten, das heißt räumlich am Grundkörper fixierten Ligand bzw. Ligandentyp aufweist. Generell sind "ein/eine" im Rahmen dieser Offenbarung als unbestimmte Artikel zu lesen, also ohne ausdrücklich gegenteilige Angabe immer auch als "mindestens ein/mindestens eine". Beispielsweise kann eine Mischung aus zwei oder mehr unterschiedliche Ligandentypen vorgesehen sein. Umgekehrt können "ein/eine" auch als "nur ein/nur eine" verstanden werden. Beispielsweise kann lediglich ein einziger Ligandtyp vorgesehen sein. Unter einem Ligand wird im Rahmen der vorliegenden Offenbarung ein Molekül verstanden, das an eine Zielstruktur spezifisch binden und/oder mit der Zielstruktur spezifisch reagieren bzw. interagieren kann, um durch beispielsweise durch Bindung, Hemmung, Deaktivierung und/oder Reaktion die biologische Funktionalität der Zielstruktur zu deaktivieren. Beispielsweise kann der Ligand hierzu die Zielstruktur enzymatisch verändern, also z. B. als Hydrolase, Isomerase, Ligase, Lyase, Transferase und/oder Oxidoreduktase wirken, oder die Zielstruktur quervernetzen oder anderweitig verändern, um so ihre biologische Funktionalität zu unterbinden. Alternativ oder zusätzlich kann der Ligand beispielsweise als Agonist oder Antagonist fungieren oder ein Paratop sein oder umfassen, mittels welchem ein Epitop einer Zielstruktur gebunden wird. Die Bindung des Liganden an die Zielstruktur kann grundsätzlich reversibel oder irreversibel sein und beispielsweise durch lonenbindungen, Wasserstoffbrückenbindungen, Van-der-Waals-Kräfte, hydrophobe Effekte oder beliebige Kombinationen hieraus bewirkt werden. Weiterhin kann der Ligand die Zielstruktur grundsätzlich unverändert binden oder chemisch modifizieren. Die Affinität eines Liganden zur Zielstruktur kann mit Hilfe von fachüblichen Ligandenbindungstests bestimmt werden. Zielstruktur des wenigstens einen Liganden ist vorliegend zumindest ein Fibrogen und/oder ein Zytokin, mit welchem das ophthalmologische Implantat im implantierten Zustand in Kontakt kommt bzw. kommen kann. Als Fibrogene werden vorliegend Stoffe und Zellen bezeichnet, die eine Fibrose hervorrufen. Als Zytokine werden vorliegend Stoffe bezeichnet, die das Wachstum und/oder die Differenzierung von Zellen im menschlichen und tierischen Auge einleiten und/oder regulieren. Auf diese Weise kann das erfindungsgemäße ophthalmologische Implantat nach seiner Implantation in ein menschliches oder tierisches Auge Fibrogene und/oder Zytokine binden bzw. ihre biologische Funktion deaktivieren, so dass die Transformation bzw. Proliferation verbleibender Linsenepithelzellen verhindert und damit das Auftreten von Fibrose, PCO und ähnlichen Erkrankungen unterbunden wird. Indem der wenigstens eine Ligand bzw. Ligandentyp am Grundkörper immobilisiert vorliegt, wird ein Verdünnen oder Herausschwemmen des Liganden beispielsweise durch Kammerwasser zuverlässig verhindert, wodurch die antifibrotische bzw. antizytokine Wirkung des erfindungsgemäßen Implantats beispielsweise auch im Unterschied zu einem Wirkstoffabgabesystem (Drug Delivery System) über einen sehr langen Zeitraum aufrecht erhalten werden kann. Erfindungsgemäß ist vorgesehen, dass zumindest ein Teil einer Oberfläche des Grundkörpers mit einem Schichtsystem beschichtet ist, welches den immobilisierten Ligand umfasst. Mit anderen Worten ist es vorgesehen, dass der Grundkörper, der seinerseits vollständig oder teilweise aus einem oder mehreren Polymeren bestehen kann, zumindest teilweise oder vollständig mit einem Schichtsystem beschichtet ist, wobei das Schichtsystem seinerseits aus einer Schicht oder aus zwei oder mehr Schichten besteht und wobei wenigstens eine der Schichten den wenigstens einen Ligand umfasst oder aus dem wenigstens einen Ligand besteht. Das Schichtsystem kann also grundsätzlich die gesamte Oberfläche des Grundkörpers oder lediglich einen oder mehrere ausgewählte Oberflächenbereiche des Grundkörpers bedecken. Ebenso kann vorgesehen sein, dass das Schichtsystem zwei Schichten umfasst, die den wenigstens einen Ligand umfassen bzw. aus diesem bestehen, wobei die wenigstens zwei Schichten die gleiche oder eine unterschiedliche Zusammensetzung besitzen können. Hierdurch können zeitlich und/oder örtlich gestaffelte Wirkeffekte realisiert werden. Erfindungsgemäß ist weiterhin vorgesehen, dass wenigstens eine der Schichten den wenigstens einen Ligand und wenigstens eine andere der Schichten ein Polymer aus der Gruppe Polyethylenimine, Polyamine und Polyallylamine umfasst. Hierdurch können oberflächengeladene Schichten hergestellt werden.

In einer vorteilhaften Ausgestaltung der Erfindung ist vorgesehen, dass das ophthalmologische Implantat als Intraokularlinse (IOL), insbesondere akkommodierende Intraokularlinse, oder als Ring, insbesondere Kapselspannring, ausgebildet ist. Hierdurch können die erfindungsgemäß realisierbaren Vorteile im Rahmen unterschiedlicher Augenoperationen und Implantattypen realisiert werden. Dies gilt in besonderem Maße für ein als akkommodierende IOL ausgebildetes Implantat, da bei einer solchen IOL eine auftretende Fibrose die Akkomodationsfähigkeit der IOL beeinträchtigen oder vollständig verhindern könnte. Weiterhin kann das ophthalmologische Implantat als Schlauch, insbesondere als Dialyseschlauch ausgebildet sein.

In einer weiteren vorteilhaften Ausgestaltung der Erfindung ist vorgesehen, dass der Ligand in einer Matrix eingebettet ist. Dies stellt eine einfache Möglichkeit zur Immobilisierung über den Mechanismus einer sterischen Hinderung dar. Die Matrix weist hierzu ein dreidimensionales Gitter mit Hohlräumen auf, in welchen Moleküle des Ligandentyps angeordnet sind, wobei die Ligandenmoleküle zu groß sind, um durch die Lücken des Gitters auszutreten. Im Gegensatz zu einem Drug Delivery System, bei welchem Wirkstoffe bewusst aus einer Matrix in die Umgebung abgegeben werden sollen, verbleibt der Ligand damit dauerhaft innerhalb der Matrix. Es versteht sich, dass die Lücken im Gitter der Matrix umgekehrt groß genug sein müssen, damit die Zielstrukturen des Liganden hindurchtreten und mit dem Ligand wechselwirken können. Um die Größe bzw. das Volumen des Liganden anzupassen, so dass dieser zuverlässig innerhalb der Matrix verbleibt, kann es vorgesehen sein, dass der Ligand mit einem entsprechend großen Molekül gekoppelt wird. Wenn der Ligand beispielsweise ein für die betreffende Matrix zu kleiner Antikörper ist, kann der Ligand beispielsweise als Fusionsprotein aus dem Antikörper und einem weiteren Protein ausgebildet sein und in der Matrix immobilisiert werden. Weiterhin kann vorgesehen sein, dass der Ligand kovalent an wenigstens ein Polymer, insbesondere an wenigstens ein Biopolymer gebunden ist. Hierdurch kann ebenfalls eine Immobilisierung in der zugeordneten Matrix erreicht werden. Alternativ kann der kovalent an das Polymer gebundene Ligand aber auch ohne Einbettung in eine Matrix verwendet werden, wenn das Polymer selbst - und damit auch der kovalent gebundene Ligand - am Grundkörper immobilisiert ist. In einer vorteilhaften Ausgestaltung der Erfindung ist es vorgesehen, dass der Grundkörper zumindest bereichsweise aus dem mit wenigstens einem Ligand kovalent gekoppelten Polymer besteht. Mit anderen Worten ist es vorgesehen, dass der Grundkörper vollständig, überwiegend oder teilweise aus dem Polymer besteht, an welches der Ligand kovalent gekoppelt ist. Unter einem Polymer werden im Rahmen der vorliegenden Offenbarung Makromoleküle verstanden, die aus einer oder mehreren Struktureinheiten (Wiederholeinheiten) aufgebaut sind. Das Polymer kann generell aus identischen oder nicht-identischen Makromolekülen bestehen und ein synthetisches oder halbsynthetisches Polymer oder ein Biopolymer sein. Unter einem Biopolymer werden im Rahmen der vorliegenden Offenbarung Polymere verstanden, die in der Zelle eines Lebewesens synthetisiert werden, sowie Polymere, die aus biogenen Rohstoffen (nachwachsenden Rohstoffen) bestehen und/oder biologisch abbaubar sind (biogene und biologisch abbaubare Polymere). Unter den Begriff Biopolymer fallen also biobasierte Biopolymere, die biologisch abbaubar oder auch nicht biologisch abbaubar sind, sowie erdölbasierte Polymere, die biologisch abbaubar sind.

In einer weiteren vorteilhaften Ausgestaltung der Erfindung ist vorgesehen, dass der wenigstens eine Ligand über einen Spacer, das heißt über eine als Abstandshalter fungierende Struktureinheit, kovalent an das wenigstens eine Polymer gekoppelt ist. Hierdurch kann einerseits der Ligand besonders einfach an die jeweils vorhandenen reaktiven Gruppen des Polymers gebunden werden, andererseits kann hierdurch die Bindungsfähigkeit des Liganden verbessert werden, da beispielsweise etwaige sterische Hinderungen, die eine Interaktion mit der Zielstruktur behindern könnten, durch Wahl eines geeigneten Spacers vermieden werden können.

In einer weiteren vorteilhaften Ausgestaltung der Erfindung ist vorgesehen, dass das wenigstens eine Polymer ein Polysaccharid aus der Gruppe Cellulose, ein Celluloseether mit Methyl- und/oder Ethyl- und/oder Propylgruppen, insbesondere Hydroxypropylmethylcellulose, Hydroxyethylmethylcellulose und/oder Methylcellulose, ein Glykosaminoglykan, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopektin, Callose, Chitosan, Polygalactomannan, Dextran, Xanthan und/oder eine Mischung und/oder ein physiologisch akzeptables Salz, insbesondere Alkalisalz, davon ist. Hierdurch können die Eigenschaften, beispielsweise die Viskoelastizität, optimal an den jeweiligen Einsatz- und Verwendungszweck des Implantats angepasst werden. Polysaccharide, die auch als Glykane bezeichnet werden, stellen generell eine Unterklasse der Kohlenhydrate dar und sind Vielfachzucker aus Monosaccharideinheiten (z. B. Glukose, Fruktose, Galactose usw.), welche eine Kette bilden. Jedes Monosaccharid, das auch als Einfachzucker bezeichnet wird, besteht aus einer Kette von Kohlenstoff-Atomen. Nach der Anzahl der Kohlenstoffatome spricht man von Triosen (3), Tetrosen (4), Pentosen (5), Hexosen (6), Heptosen (7) usw. Die Polysaccharide können nach Art ihrer Saccharideinheiten in Homoglykane mit nur einer Art Einfachzucker und Heteroglykane mit zwei oder mehr verschiedenen Arten von Einzelzuckern eingeteilt werden. Weiterhin können Polysaccharide unsubstituiert oder substituiert sein und eine oder mehrere Seitengruppen wie beispielsweise Hydroxyl-, Carboxy-, Amino- oder Sulfatgruppen tragen.

In einer weiteren vorteilhaften Ausgestaltung der Erfindung ist vorgesehen, dass das ophthalmologische Implantat einen Grundkörper mit wenigstens einem haptischen und wenigstens einem optischen Teil umfasst, wobei der wenigstens eine Ligand vorzugsweise zumindest am haptischen Teil angeordnet ist. Vorzugsweise ist der Ligand vollständig im bzw. am haptischen Teil angeordnet. Hierdurch wird zuverlässig verhindert, dass die Funktionalität des optischen Teils durch den Ligand und etwaige weitere Verbindungen beeinträchtigt wird. Darüber hinaus besteht die Möglichkeit, den haptischen Teil als physikalische Barriere gegen Zellwanderung zu gestalten, indem er im implantierten Zustand des ophthalmologischen Implantats einen vorbestimmten Druck auf das umgebende Gewebe, beispielsweise auf den Kapselsack ausübt. Ein gebogener haptischer Teil ermöglicht damit einen signifikant erhöhten Kontakt mit dem umgebenden Gewebe und verhindert damit zusätzlich ein Unterwandern mit Zellen. Obwohl der Grundkörper in Abhängigkeit von der Ausgestaltung des ophthalmologischen Implantats grundsätzlich auch einteilig sein kann, bietet die Ausgestaltung mit einem haptischen und einem optischen Teil den weiteren Vorteil, dass der Grundkörper aus unterschiedlichen Materialien hergestellt sein kann.

Alternativ oder zusätzlich kann vorgesehen sein, dass der Grundkörper einen Aufnahmebereich umfasst, in welchem der immobilisierte Ligand angeordnet ist. Beispielsweise kann der Grundkörper eine Tasche oder dergleichen aufweisen, innerhalb welcher der Ligand immobilisiert vorliegt. Hierdurch kann der Ligand optimal in Abhängigkeit von dem jeweiligen Einsatzzweck des Implantats angeordnet werden. Alternativ oder zusätzlich kann der wenigstens eine Ligand kovalent an den Grundkörper gebunden sein. Diese mittelbare oder unmittelbare Anbindung stellt eine besonders zuverlässige Immobilisierung sicher. Beispielsweise kann zur kovalenten Bindung ein Quervernetzer wie beispielsweise 1,4-Butandioldiglycidylether verwendet werden. In Abhängigkeit von dem jeweiligen Grundkörper-Material können aber natürlich auch andere geeignete Quervernetzer oder Gemische davon verwendet werden.

Weitere Vorteile ergeben sich, indem der wenigstens eine Ligand zumindest ein im Kammerwasser vorkommendes Fibrogen und/oder Zytokin, insbesondere TGF-β, TNF-α und/oder Interleukin-1, bindet und/oder deaktiviert. Hierdurch können die nachteiligen Effekte von in Kammerwasser vorkommenden Fibrogenen bzw. Zytokinen therapeutisch wirksam vermieden werden. Der Ausdruck "TGF-β" umfasst im Rahmen der vorliegenden Offenbarung implizit auch die Isoformen TGF-β1, TGF-β2, TGF-β3 und TGF-β4. Die Gehalte an Fibrogenen bzw. Zytokinen im Kammerwasser erhöhen sich nach einem chirurgischen Trauma durch die Implantation des Implantats und fallen in der Regel erst nach Monaten wieder auf ihre Ausgangswerte ab. Durch die dauerhafte Entfernung von Fibrogenen bzw. Zytokinen über den Liganden können die nachteiligen Effekte dieser Stoffe über den geforderten, vergleichsweise langen Zeitraum von mehreren Monaten zuverlässig verhindert werden, da beispielsweise keine stabile Transdifferenzierung der Epithelzellen in fibroblastenartige Zellen, die PCO und Fibrose verursachen, möglich ist. Dies ist besonders wichtig für akkommodative IOLs, deren Funktionalität durch Fibrose besonders stark beeinträchtigt werden kann.

In einer weiteren vorteilhaften Ausgestaltung der Erfindung ist vorgesehen, dass der wenigstens eine Ligand ausgewählt ist aus einer Gruppe, die Antikörper, insbesondere anti-TGF-β-Antikörper, anti-TGF-α-Antikörper und/oder anti-Interleukin-1-Antikörper, Fab-Fragmente, variable Einzelkettenfragmente (scFv) und multivalente Antikörperfragmente (scFv Multimere) umfasst. Hierdurch kann eine optimal auf das zu entfernende Fibrogen bzw. Zytokin angepasste Selektionierung durchgeführt werden. Antikörperfragmente bieten den Vorteil einer hohen Bindungsaffinität bzw. -avidität und Spezifität für ein breites Spektrum an Zielstrukturen und Haptenen. Einzelkettenfragmente können zudem als Diabodies (60 kDa), Triabodies (90 kDa), Tetrabodies (120 kDa) etc. vernetzt bzw. exprimiert werden, wobei unterschiedliche Linkerlängen zwischen V-Domänen möglich sind. Ein besonderer Vorteil ist zudem, dass Moleküle von 60-120 kDa die Penetration von Zellen erhöhen und schnellere Clearance-Raten als korrespondierende Igs (150 kDa) besitzen. Es kann weiterhin vorgesehen sein, dass Ligand ein Diabody, Triabody, Tetrabody, Pentabody, Hexabody, Heptabody oder Octabody ist. Mit anderen Worten ist es vorgesehen, dass Ligand mono-, bi-, tri-, quad-, pent-, hex-, hept-, oct-, enn- oder multispezifisch ausgebildet ist. Dies erlaubt die Quervernetzung von zwei, drei, vier, fünf, sechs, sieben oder acht Zielstrukturen bzw. Zielproteinen, wobei scFv Multimere besonders präzise und individuell an die gegebenenfalls patientenspezifische räumliche Anordnung der Zielepitope bestimmter Fibrogene bzw. Zytokine angepasst werden können. Die erhöhte Bindungswertigkeit von scFv-Multimeren führt zu einer besonders hohen Avidität.

Ein zweiter Aspekt der Erfindung betrifft ein Verfahren gemäß Anspruch 9 zum Herstellen eines ophthalmologischen Implantats, bei welchem ein Grundkörper mit wenigstens einem am Grundkörper immobilisierten Ligand, mittels welchem im implantierten Zustand des ophthalmologischen Implantats zumindest ein Fibrogen und/oder Zytokin gebunden und/oder deaktiviert werden kann, hergestellt wird. Hierdurch kann ein ophthalmologisches Implantat geschaffen werden, welches das Risiko von PCO und Fibrose verringert. Erfindungsgemäß ist es vorgesehen, dass auf einem Oberflächenbereich des Grundkörpers ein Schichtsystem mit mindestens zwei Schichten erzeugt wird, wobei wenigstens eine der Schichten den immobilisierten Ligand und wenigstens eine andere der Schichten ein Polymer aus der Gruppe Polyethylenimine, Polyamine und Polyallylamine umfasst. Mit anderen Worten wird auf einem oder mehreren Bereichen der Oberfläche des Grundkörpers oder auf dem gesamten Grundkörper ein Schichtsystem aus zwei oder mehr Schichten erzeugt, wobei mindestens eine Schicht den Ligand, der beispielsweise kovalent mit einem Polysaccharid oder einem anderen Polymer gekoppelt sein kann, umfasst bzw. aus diesem besteht und mindestens eine weitere Schicht ein anderes Polymer aus der Gruppe Polyethylenimine, Polyamine und Polyallylamine umfasst bzw. aus einem der genannten Polymere oder einer Mischung daraus besteht. Vorzugsweise besitzt das Schichtsystem eine Sandwichsstruktur mit mehreren abwechselnden Schichten aus dem gegebenenfalls mit einem Polysaccharid gekoppelten Ligand und dem anderen Polymer. Vorzugsweise besteht die Deckschicht des Schichtsystems aus dem Ligand bzw. einem kovalent mit dem Ligand gekoppelten Polymer, insbesondere einem Polysaccharid. Weitere sich hieraus ergebenden Merkmale und deren Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

In einer vorteilhaften Ausgestaltung der Erfindung hat es sich als vorteilhaft gezeigt, dass der wenigstens eine Ligand kovalent an ein Polysaccharid gekoppelt wird. Zum kovalenten Koppeln wird dabei vorzugsweise ein Verfahren durchgeführt, das zumindest die Schritte: Bereitstellen des Liganden, wobei dieser wenigstens eine Aminogruppe aufweist, Bereitstellen des Polysaccharids, wobei dieses wenigstens eine Carbonsäuregruppe aufweist, zumindest partielles Aktivieren der Carbonsäuregruppe des Polysaccharids und Koppeln der aktivierten Carbonsäuregruppe mit der wenigstens einen Aminogruppe des Liganden umfasst. Hierdurch können insbesondere Antikörper, Antikörperfragmente und dergleichen, die regelmäßig mehrere Aminogruppen aufweisen, mit dem Carbonsäuregruppen-enthaltenden Polysaccharid kovalent verbunden werden. Die teilweise oder vollständige Aktivierung der vorhandenen Carbonsäuregruppe(n) des Polysaccharids kann beispielsweise mit Hilfe von N-Hydroxysulfosuccinimid (NHS/Sulfo-NHS) erfolgen, so dass in wässriger Lösung eine gegebenenfalls Carbodiimid-vermittelte (z. B. 1-Ethyl-3-(-3-dimethylaminopropyl)carbodiimidhydrochlorid/EDC und/oder N', N'-dicyclohexylcarbodiimid/DCC) kovalente Kopplung bei vorzugsweise physiologischen pH-Werten erfolgen kann.

In einer weiteren vorteilhaften Ausgestaltung der Erfindung ist vorgesehen, dass der Grundkörper mit einem Wirkstoffabgabesystem versehen wird, welches dazu ausgebildet ist, im implantierten Zustand des ophthalmologischen Implantats Alkaliionen aus der Gruppe Li⁺, K⁺, Rb⁺ und/oder Cs⁺ abzugeben. Mit anderen Worten ist es vorgesehen, dass das erfindungsgemäße ophthalmologische Implantat bzw. sein Grundkörper zusätzlich zu dem immobilisierten Ligand mit einem Wirkstoffabgabesystem, das im Unterschied zum immobilisierten Ligand auch als Drug Delivery System bezeichnet werden kann, für Alkaliionen mit Ausnahme von Na⁺-Ionen (und radioaktiven Fr⁺-Ionen) versehen ist. Alkaliionen aus der Gruppe Li⁺, K⁺, Rb⁺ und Cs⁺ können das Entstehen von PCO und Fibrose zusätzlich erheblich verlangsamen oder sogar vollständig verhindern, wobei die Wirksamkeit generell bei Li⁺ am höchsten ist. Natrium-Ionen besitzen nach derzeitiger Erkenntnis keinerlei Wirksamkeit und können das Entstehen von PCO und Fibrose nicht verhindern. Unter dem Begriff "Alkaliionen" werden daher im Rahmen der vorliegenden Offenbarung generell Li⁺, K⁺, Rb⁺ und Cs⁺, aber nicht Na⁺- und Fr⁺-Ionen verstanden, sofern Na⁺- bzw. Fr⁺-Ionen nicht explizit genannt werden. Die genannten Alkaliionen Li⁺, K⁺, Rb⁺ und Cs⁺ können dabei grundsätzlich einzeln oder in beliebiger Kombination verwendet werden. Beispielsweise kann das Wirkstoffabgabesystem lediglich Li⁺-Ionen oder lediglich Rb⁺-Ionen abgeben. Ebenso kann vorgesehen sein, dass das Wirkstoffabgabesystem zur Abgabe von Li⁺-Ionen und von K⁺-Ionen ausgebildet ist und so weiter. Alkaliionen der genannten Gruppe besitzen den Vorteil, dass sie - einzeln und in beliebiger Kombination - in den zur Verhinderung von PCO und Fibrose benötigten Konzentrationen keine oder nur eine sehr geringe Toxizität insbesondere für Augengewebe besitzen. Ohne auf diese Theorie festgelegt werden zu wollen, wird davon ausgegangen, dass topisch verabreichte Alkaliionen aus der genannten Gruppe in der Lage sind, die Umwandlung von äquatorialen Epithelzellen, die im Rahmen einer Augenoperation nicht entfernt wurden, in Fibroplasten zu verhindern, wobei Fibroplasten wie bereits erwähnt wurde die Hauptursache für PCO und Fibrose sind. Die Wirkung basiert dabei vermutlich auf der Fähigkeit von Alkaliionen der genannten Gruppe, den ruhenden, polarisierten Linsenepithelzell-Phänotyp zu stabilisieren. Die betreffenden Epithelzellen behalten daher die für Linsenepithelzellen charakteristische apikal-basolaterale Polarität und Kopfsteinpflaster-artige Anordnung bei. Die Alkaliionen der genannten Gruppe blockieren bei örtlicher Anwendung auch den potenten epithelialen Mesenchymale Transition (EMT)-fördernden Effekt des transformierenden Wachstumsfaktors Beta (TGF-β) auf Linsenepithelzellen. Wie im Rahmen von Versuchen festgestellt wurde, dissoziierten in TGF-β-behandelten Explantaten Zellen progressiv voneinander weg und nahmen verschiedene längliche Spindelformen an. Weiterhin exprimierten diese Zellen in erheblichem Umfang α-SMA (Alpha-actin-2, Alpha Smooth Muscle Actin). Diese Merkmale sind charakteristisch für die Entstehung von PCO. Durch die Behandlung mit Alkaliionen der genannten Gruppe konnten die Bildung von α-SMA effektiv blockiert und die Zellen in der polarisierten, anhaftenden, Kopfsteinpflaster-artigen Monoschicht stabilisiert werden. Als Gegenionen (Anion) zu dem oder den verwendeten Alkaliionen (Kation) können grundsätzlich alle geeigneten bzw. pharmazeutisch akzeptablen, aus Atomen und/oder Molekülen entstandenen Anionen verwendet werden. Besonders bevorzugt sind Chlorid-Ionen (CI⁻). Das Wirkstoffabgabesystem kann generell ausschließlich aus einer Alkaliionen der genannten Gruppe enthaltenden Verbindung bestehen. Alternativ können weitere Komponenten, beispielsweise zur Einstellung der Wirkstoffabgabe oder als Binde- oder Trennmittel, vorgesehen sein. Weiterhin kann das Wirkstoffabgabesystem eine Schicht oder Teil einer Schicht eines Schichtsystems sein.

Ein dritter Aspekt der Erfindung betrifft eine Verwendung eines Liganden, mittels welchem im implantierten Zustand eines ophthalmologischen Implantats zumindest ein Fibrogen und/oder Zytokin zu binden und/oder zu deaktivieren ist, zur Herstellung eines ophthalmologischen Implantats gemäß dem ersten Erfindungsaspekt. Hierdurch kann ein ophthalmologisches Implantat geschaffen werden, welches das Risiko von PCO und Fibrose verringert. Weitere sich hieraus ergebenden Merkmale und deren Vorteile sind den Beschreibungen des ersten und zweiten Erfindungsaspekts zu entnehmen, wobei vorteilhafte Ausgestaltungen des ersten und zweiten Erfindungsaspekts als vorteilhafte Ausgestaltungen des dritten Erfindungsaspekts und umgekehrt anzusehen sind.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische und ausschnittsweise Seitenansicht eines aus dem Stand der Technik bekannten ophthalmologischen Implantats;
- Fig. 2: eine vergrößerte Darstellung des in Fig. 1 gezeigten Detailbereichs II;
- Fig. 3: eine vergrößerte Darstellung des in Fig. 1 gezeigten Detailbereichs III;
- Fig. 4: eine Prinzipdarstellung einer kovalenten Kopplungsreaktion eines Liganden an ein Polysaccharid;
- Fig. 5: eine chemische Formel des mit dem Ligand gekoppelten Polysaccharids;
- Fig. 6: eine schematische seitliche Schnittansicht eines Ausführungsbeispiels eines erfindungsgemäßen ophthalmologischen Implantats; und
- Fig. 7: eine schematische und ausschnittsweise Ansicht des erfindungsgemäßen ophthalmologischen Implantats, an welchem das mit dem Ligand gekoppelte Polysaccharid immobilisiert ist.

### Bevorzugte Ausführung der Erfindung

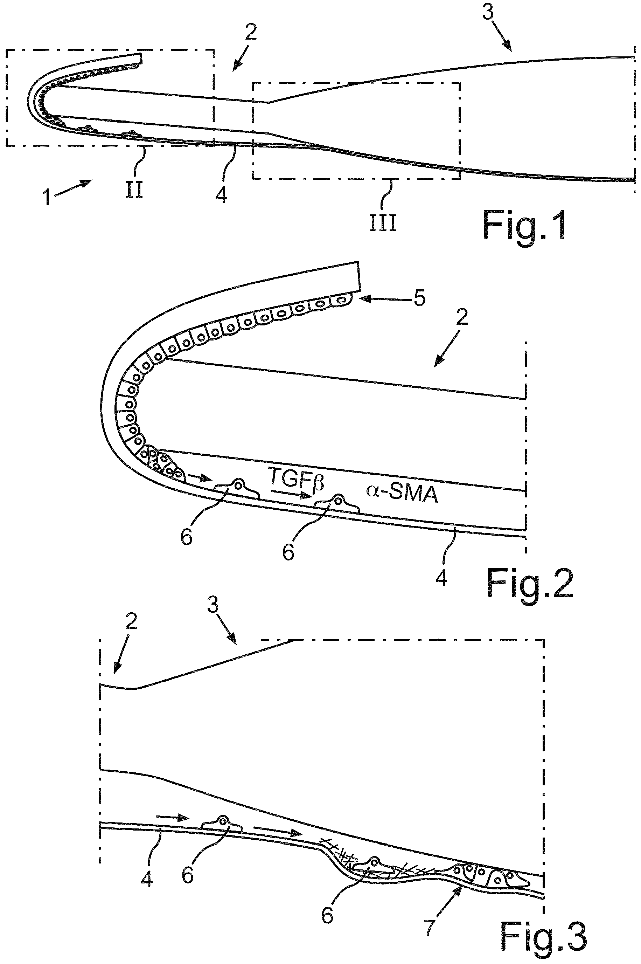

Fig. 1 zeigt eine schematische und ausschnittsweise Seitenansicht eines aus dem Stand der Technik bekannten ophthalmologischen Implantats 1, welches vorliegend als Intraokularlinse ausgebildet ist. Das Implantat 1 umfasst in an sich bekannter Weise einen haptischen Teil 2 und einen optischen Teil 3. Zum besseren Verständnis zeigt Fig. 2 eine vergrößerte Darstellung des in Fig. 1 gezeigten Detailbereichs II, während Fig. 3 eine vergrößerte Darstellung des in Fig. 1 gezeigten Detailbereichs III zeigt. Man erkennt, dass das Implantat 1 insbesondere im Bereich des haptischen Teils 2 nicht durchgehend bündig am posterioren Kapselsack 4 anliegt, da der Kapselsack 4 Falten und Hohlräume bildet. Dies führt dazu, wie in Fig. 2 schematisch dargestellt ist, dass sogenannte E-Zellen 5, die im Äquatorialbereich des Kapselsacks 4 sitzen, mitotisch aktiv sind und normalerweise eine Kopfsteinpflaster-artige Monoschicht bilden, unter dem Einfluss des transformierenden Wachstumsfaktors Beta (TGF-β) progressiv voneinander wegdissoziieren und verschiedene längliche Spindelformen 6 annehmen. Weiterhin exprimierten diese spindelförmigen Zellen 6 in erheblichem Umfang α-SMA (Alpha-actin-2, Alpha Smooth Muscle Actin), was zur Entstehung einer posterioren Kapselopazifikation (posterior capsule opacification, PCO, Cataracta secundaria) führt. Man erkennt in Fig. 3, dass die spindelförmigen Zellen 6, die zu Fibrose und Faltenbildung führen, dabei auch den optischen Teil 3 des Implantats 1 unterwandern. Mitunter bilden sich sogenannte Wedl-Zellen 7, die aufgeblähte, ungleichmäßig geformte Fasern bilden, reißen und Zelltrümmer verbreiten. Diese anomalen Wedl-Zellen 7 und deren Bruchstücke sammeln sich in ungeordneter Weise auch am optischen Teil 3 und bilden Trübungszentren aus. Die Wedl-Zellen 7 entsprechen histopathologisch den klinisch sichtbaren Hirschberg-Elschnig-Perlen, die auch für die Bildung des sogenannten Soemmering-Rings, der zuerst in Verbindung mit Augentraumata beschrieben wurde, verantwortlich sind.

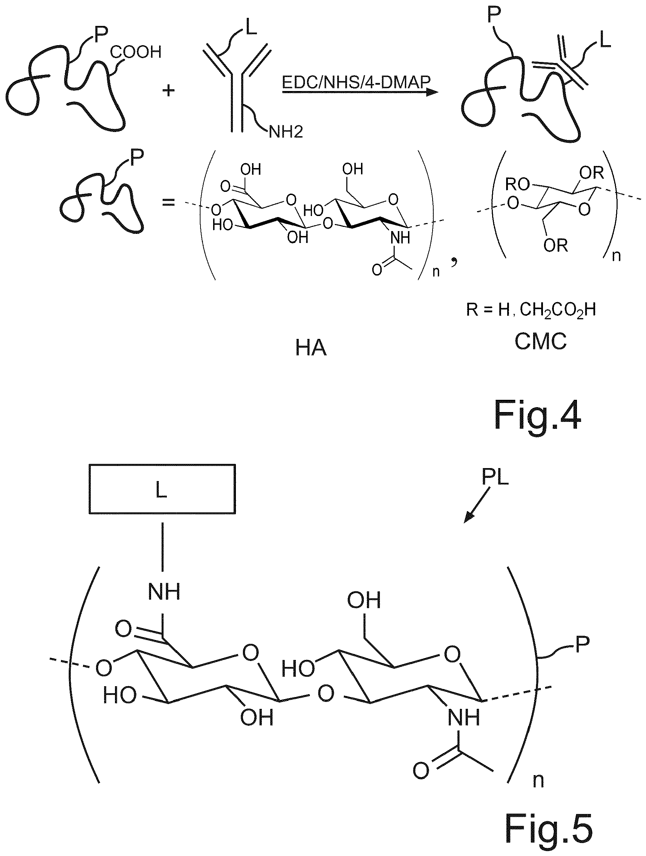

Fig. 4 zeigt eine Prinzipdarstellung einer kovalenten Kopplungsreaktion eines Liganden L an ein Polymer bzw. Biopolymer, bei welchem es sich vorliegend exemplarisch um ein Polysaccharid P, nämlich um Hyaluronsäure HA oder Carboxymethylcellulose CMC handelt. Anstelle des Polysaccharids P kann der Ligand L grundsätzlich auch mit anderen Polymeren gekoppelt werden. Hyaluronsäure gehört zu der Gruppe der Glykosaminoglykane, bei denen es sich um Polysaccharide handelt, die aus repetitiven, linear aufgebauten und sauren Disaccharid-Einheiten bestehen. Die Disaccharid-Einheiten von Glykosaminoglykanen bestehen generell aus Estern einer Uronsäure. Meist handelt es sich um Glucuronsäure, seltener um Iduronsäure. Die Disaccharid-Einheiten sind 1-3-glykosidisch mit einem Aminozucker (z.B. N-Acetylglucosamin) verbunden. Die Kettenbildung der Disaccharid-Einheiten erfolgt durch 1-4-glykosidische Bindungen. Durch entsprechende Seitengruppen (Hydroxyl-, Carboxy- oder Sulfatgruppen) sind die Glykosaminoglykane negativ geladen. Glykosaminoglykane können in sulfatierte und nicht-sulfatierte Glykosaminoglykane eingeteilt werden. Hyaluronsäure (Hyaluronan) ist das einzige nicht-sulfatierte Glykosaminoglykan und weist freie Carboxy-Gruppen auf, die durch Aktivierung mit Hilfe von N-Hydroxysulfosuccinimid (NHS/Sulfo-NHS) und anschließende Carbodiimid-vermittelte (z. B. 1-Ethyl-3-(-3-dimethylaminopropyl)carbodiimidhydrochlorid/EDC und/oder N', N'-dicyclohexylcarbodiimid/DCC) Reaktion kovalent mit dem Ligand L gekoppelt werden kann. Gegebenenfalls kann 4-(Dimethylamino)-pyridin (4-DMAP) als nucleophiler Katalysator verwendet werden, um sterisch anspruchsvolle Liganden L unter milden Bedingungen zu koppeln. Die kovalente Kopplung kann analog mit Säuregruppen der Carboxymethylcellulose durchgeführt werden.

Als Ligand L wird im in Fig. 4 gezeigten Ausführungsbeispiel exemplarisch ein anti-TGF-β-Antikörper verwendet, der über eine freie Aminogruppe an das Polysaccharid P gekoppelt wird. Alternativ kann als Ligand L beispielsweise auch ein anti-TGF-α-Antikörper, ein anti-Interleukin-1-Antikörper, ein Fab-Fragment, ein variables Einzelkettenfragment (scFv) oder ein multivalentes Antikörperfragment (scFv Multimer) sowie beliebige Kombinationen hieraus verwendet und mit dem Polysaccharid P gekoppelt werden.

Das resultierende, mit dem Ligand L gekoppelte Polysaccharid P, ist schematisch in Fig. 5 gezeigt, wobei exemplarisch Hyaluronsäure als Polysaccharid P und ein Antikörper, beispielsweise Anti-TGF-β und/oder Anti-IL-1α, als Ligand L dargestellt sind. Die Polysaccharid-Ligand-Verbindung PL kann zur Entfernung und/oder biologischen Deaktivierung von Fibrogenen und/oder Zytokinen, mit welchen ein erfindungsgemäßes ophthalmologisches Implantat 10 im implantierten Zustand in Kontakt kommt bzw. kommen kann, verwendet werden. Es kann vorgesehen sein, dass die Polysaccharid-Ligand-Verbindung PL als physiologisch akzeptables Salz, insbesondere als Lithiumsalz vorliegt. Hierzu kann beispielsweise Lithiumhyaluronat als Edukt verwendet und mit dem Ligand L gekoppelt werden. Alternativ kann die Polysaccharid-Ligand-Verbindung PL nach der Herstellung in ein physiologisch akzeptables Salz, beispielsweise ein Lithiumsalz umgewandelt werden.

Ohne auf diese Theorie festgelegt werden zu wollen, wird davon ausgegangen, dass topisch verabreichte Alkaliionen aus der Gruppe Li⁺, K⁺, Rb⁺ und Cs⁺ das Entstehen von PCO und Fibrose erheblich verlangsamen oder sogar vollständig verhindern können, wobei die Wirksamkeit generell bei Li⁺ am höchsten ist. Die genannten Ionen verhindern die Umwandlung von äquatorialen Epithelzellen, die im Rahmen einer Augenoperation nicht entfernt wurden, in Fibroplasten, wobei Fibroplasten die Hauptursache für PCO und Fibrose sind. Die Wirkung basiert dabei vermutlich auf der Fähigkeit von Alkaliionen der genannten Gruppe, den ruhenden, polarisierten Linsenepithelzell-Phänotyp zu stabilisieren. Die betreffenden Epithelzellen behalten daher die für Linsenepithelzellen charakteristische apikal-basolaterale Polarität und Kopfsteinpflaster-artige Anordnung bei. Die Alkaliionen der genannten Gruppe blockieren bei örtlicher Anwendung auch den potenten epithelialen Mesenchymale Transition (EMT)-fördernden Effekt des transformierenden Wachstumsfaktors Beta (TGF-β) auf Linsenepithelzellen. Wie im Rahmen von Versuchen festgestellt wurde, dissoziierten in TGF-β-behandelten Explantaten Zellen progressiv voneinander weg und nahmen verschiedene längliche Spindelformen an. Weiterhin exprimierten diese Zellen in erheblichem Umfang α-SMA (Alpha-actin-2, Alpha Smooth Muscle Actin). Diese Merkmale sind charakteristisch für die Entstehung von PCO. Durch die Behandlung mit Alkaliionen der genannten Gruppe konnten die Bildung von α-SMA effektiv blockiert und die Zellen in der polarisierten, anhaftenden, Kopfsteinpflaster-artigen Monoschicht stabilisiert werden. Als Gegenionen (Anion) zu dem oder den verwendeten Alkaliionen (Kation) können grundsätzlich alle geeigneten bzw. pharmazeutisch akzeptablen, aus Atomen und/oder Molekülen entstandenen Anionen verwendet werden. Besonders bevorzugt sind Chlorid-Ionen (CI⁻). Das Wirkstoffabgabesystem kann generell ausschließlich aus einer Alkaliionen der genannten Gruppe enthaltenden Verbindung bestehen. Alternativ können weitere Komponenten, beispielsweise zur Einstellung der Wirkstoffabgabe der Alkaliionen oder als Binde- oder Trennmittel, vorgesehen sein.

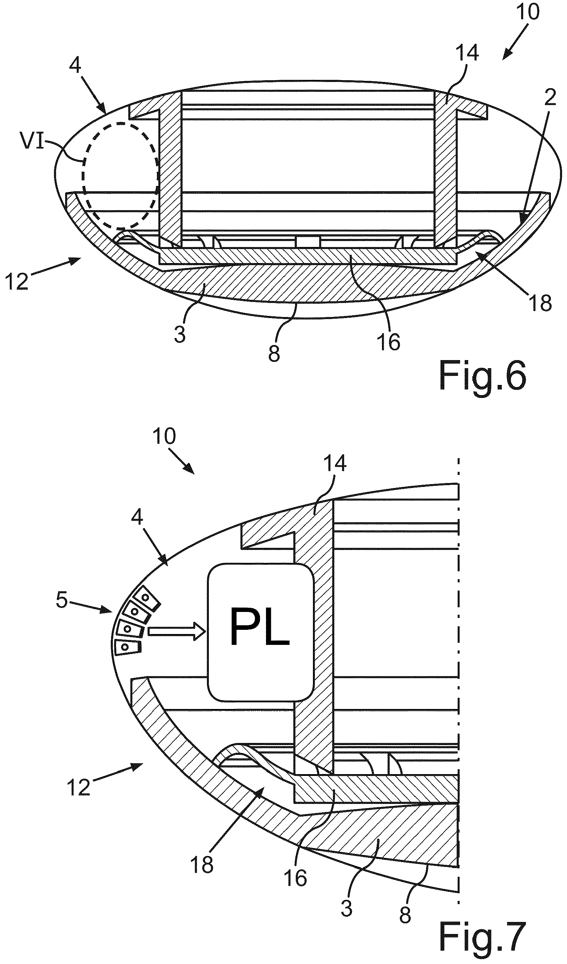

Fig. 6 zeigt eine schematische seitliche Schnittansicht eines Ausführungsbeispiels eines erfindungsgemäßen ophthalmologischen Implantats 10, das in einem Kapselsack 4 eines Patienten implantiert ist. Das Implantat 10 ist vorliegend als akkommodierende Intraokularlinse (IOL) ausgebildet und umfasst einen Grundkörper 12 mit einem haptischen Teil 2 sowie einem optischen Teil 3, der einen Linsenkörper 8 aufweist. Weiterhin umfasst das Implantat 10 einen hohlen, zentral angeordneten Zylinder 14, der im implantierten Zustand eine federnd gelagerte Membran 16 axial bezüglich einer Mittelachse des Implantats 10 mit einer Kraft beaufschlägt. Die Membran 16 ist Teil einer Wand eines gasgefüllten Hohlraums 18 und begrenzt den Hohlraum 18 in Richtung des Zylinders 14 hin, während der optische Teil 3 die dem Zylinder 14 abgewandte Seite des Hohlraums 18 begrenzt.

Man erkennt, dass sich in axialer Richtung zwischen dem haptischen Teil 2 und dem Rand des Zylinders 14 ein mit einem Kreis VI gekennzeichneter, umlaufender Aufnahmebereich befindet, in welchen äquatoriale Linsenepithelzellen (E-Zellen) 5 eindringen können und durch Sekretion von Zytokinen und/oder Fibrogenen, beispielsweise von TGF-β, Interleukin-1 und dergleichen, eine Zellvermehrung, Zelldifferenzierung und Unterwanderung des Implantats 10 mit Fibroplasten und dergleichen verursachen können, was in weiterer Konsequenz zu PCO bzw. Fibrose führen kann.

Um das Auftreten von PCO und Fibrose zu verhindern, wird die Polysaccharid-Ligand-Verbindung PL am ophthalmologischen Implantat 10 immobilisiert. Fig. 7 zeigt hierzu eine schematische und ausschnittsweise Ansicht des ophthalmologischen Implantats 10, wobei die Polysaccharid-Ligand-Verbindung PL im Aufnahmebereich VI immobilisiert ist, um Zytokine und/oder Fibrogene im Kammerwasser zu binden bzw. biologisch zu deaktivieren. Die Polysaccharid-Ligand-Verbindung PL ist dabei so angeordnet, dass die Akkomodationsfähigkeit des Implantats 10 nicht behindert wird. Vorzugsweise ist die Polysaccharid-Ligand-Verbindung PL umlaufend am Außenumfang des Zylinders 14 und damit in der Nähe der E-Zellen 5 angeordnet, um einen besonders zuverlässigen und lang anhaltenden Schutz sicherzustellen. Alternativ ist die Polysaccharid-Ligand-Verbindung PL an einem oder mehreren diskreten Orten auf der Außenseite des Implantats 10 angeordnet. Ebenfalls denkbar ist es, dass das Implantat 10 teilweise oder vollständig mit der Polysaccharid-Ligand-Verbindung PL beschichtet ist.

Es ist erfindungsgemäß ein Schichtsystem (nicht gezeigt) mit zwei oder mehr Schichten vorgesehen. Unabhängig davon ist es auch möglich, das Implantat 10 zumindest teilweise aus der Polysaccharid-Ligand-Verbindung PL auszubilden. Optional können neben der Polysaccharid-Ligand-Verbindung PL eine oder mehrere weitere Verbindungen vorgesehen sein, beispielsweise um zusätzliche medizinische Wirkungen zu erzielen, um die chemischen und/oder mechanischen Eigenschaften anzupassen und/oder um die Immobilisierung an das Implantat 10 zu bewirken oder zu unterstützen. Beispielsweise kann das Polysaccharid P über 1,4-Butandioldiglycidylether als Quervernetzer an das Material des Implantats 10 gebunden werden. Die Quervernetzungsrate kann dabei zur Kontrolle oder Einstellung der Ligandenmenge und damit zur Kontrolle der theoretisch bindbaren und/oder deaktivierbaren Menge an Fibrogenen/Zytokinen sowie zur Kontrolle der biologischen Abbaubarkeit des Polysaccharids P (z. B. durch Hyaluronidase) verwendet werden

Im nicht-implantierten Zustand kann generell vorgesehen sein, dass die Polysaccharid-Ligand-Verbindung PL nicht-hydratisiert bzw. wasserfrei, insbesondere lyophilisiert ist, um das Implantat 10 möglichst stark komprimieren und durch eine entsprechend kleine Inzision implantieren zu können. Sowohl Polysaccharide P oder andere Polymere als auch Linganden L wie etwa Antikörper sind beispielsweise durch Lyophilisieren wasserfrei erhältlich und in dieser Form auch als kovalentes Konjugat lange lagerbar. Die Hydratation mit entsprechender Volumenvergrößerung erfolgt dann automatisch nach der Implantation durch den Kontakt mit Kammerwasser. Die kovalente Bindung des Liganden L sowie die Immobilisierung des Polysaccharids P an das Implantat 10 verhindern, dass das Kammerwasser den Liganden L bzw. die Polysaccharid-Ligand-Verbindung PL auswäscht.

Alternativ oder zusätzlich kann es grundsätzlich vorgesehen sein, dass das Polysaccharid und/oder eine andere Polymerart, vorzugsweise in Form eines Hydrogels oder eines Schaums, eine - insbesondere biologisch abbaubare - Matrix bildet, in welcher der Ligand L oder mehrere unterschiedliche Liganden L eingebettet und dadurch in der Matrix immobilisiert ist bzw. sind. Die andere Polymerart kann beispielsweise ein Homo- oder Copolymer sein, welches gegebenenfalls quervernetzt ist. Beispielsweise kann die andere Polymerart ein Alginat sein. Alternativ oder zusätzlich kann die Matrix ein Alkalisalz, beispielsweise Lithiumhyaluronat, enthalten oder daraus bestehen. Hierdurch kann zusätzlich zum immobilisierten Ligand L eine Art Wirkstoffabgabesystem realisiert werden, welches über einen einstellbaren Zeitraum Zytokine und/oder Fibrogene aus dem Kammerwasser binden bzw. deaktivieren kann. Weiterhin kann es vorgesehen sein, dass auch der in der Matrix immobilisierte Ligand L mit der Zeit durch Abbau der Matrix freigesetzt wird.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

### Bezugszeichenliste

- 1: Implantat (Stand der Technik)
- 2: haptischer Teil
- 3: optischer Teil
- 4: Kapselsack
- 5: E-Zellen
- 6: Zellen
- 7: Wedl-Zellen
- 10: erfindungsgemäßes Implantat
- 12: Grundkörper
- 14: Zylinder
- 16: Membran
- 18: Hohlraum
- VI: Aufnahmebereich
- P: Polysaccharid
- L: Ligand
- PL: Polysaccharid-Ligand-Verbindung
- HA: Hyaluronsäure
- CMC: Carboxymethylcellulose

## Patentansprüche

1. Ophthalmologisches Implantat (10) mit einem Grundkörper (12) und wenigstens einem am Grundkörper (12) immobilisierten Ligand (L), mittels welchem im implantierten Zustand des ophthalmologischen Implantats (10) zumindest ein Fibrogen und/oder Zytokin zu binden und/oder zu deaktivieren ist, wobei zumindest ein Teil einer Oberfläche des Grundkörpers (12) mit einem Schichtsystem beschichtet ist, welches den immobilisierten Ligand (L) umfasst, wobei das Schichtsystem wenigstens zwei Schichten umfasst und wobei wenigstens eine der Schichten den immobilisierten Ligand (L) und wenigstens eine andere der Schichten ein Polymer aus der Gruppe Polyethylenimine, Polyamine und Polyallylamine umfasst.

2. Ophthalmologisches Implantat (10) nach Anspruch 1,
**dadurch gekennzeichnet, dass**
der Ligand (L) in einer Matrix eingebettet ist und/oder dass der Ligand (L) kovalent an wenigstens ein Polymer, insbesondere an wenigstens ein Biopolymer gebunden ist.

3. Ophthalmologisches Implantat (10) nach Anspruch 2,
**dadurch gekennzeichnet, dass**
der wenigstens eine Ligand (L) über einen Spacer kovalent an das wenigstens eine Polymer gekoppelt ist.

4. Ophthalmologisches Implantat (10) nach Anspruch 2 oder 3,
**dadurch gekennzeichnet, dass**
das wenigstens eine Polymer ein Polysaccharid (P) aus der Gruppe Cellulose, ein Celluloseether mit Methyl- und/oder Ethyl- und/oder Propylgruppen, insbesondere Hydroxypropylmethylcellulose, Hydroxyethylmethylcellulose und/oder Methylcellulose, ein Glykosaminoglykan, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopektin, Callose, Chitosan, Polygalactomannan, Dextran, Xanthan und/oder eine Mischung und/oder ein physiologisch akzeptables Salz, insbesondere Alkalisalz, davon ist.

5. Ophthalmologisches Implantat (10) nach einem der Ansprüche 1 bis 4,
**dadurch gekennzeichnet, dass**
dieses einen Grundkörper (12) mit wenigstens einem haptischen und wenigstens einem optischen Teil (2, 3) umfasst, wobei der Ligand (L) vorzugsweise zumindest am haptischen Teil (2) angeordnet ist.

6. Ophthalmologisches Implantat (10) nach einem der Ansprüche 1 bis 5,
**dadurch gekennzeichnet, dass**
der Grundkörper (12) einen Aufnahmebereich (VI) umfasst, in welchem der immobilisierte Ligand (L) angeordnet ist, und/oder dass der Ligand (L) kovalent an den Grundkörper (12) gebunden ist.

7. Ophthalmologisches Implantat (10) nach einem der Ansprüche 1 bis 6,
**dadurch gekennzeichnet, dass**
der wenigstens eine Ligand (L) zumindest ein im Kammerwasser vorkommendes Fibrogen und/oder Zytokin, insbesondere TGF-β, TNF-α und/oder Interleukin-1, bindet und/oder deaktiviert.

8. Ophthalmologisches Implantat (10) nach einem der Ansprüche 1 bis 7,
**dadurch gekennzeichnet, dass**
der wenigstens eine Ligand (L) ausgewählt ist aus einer Gruppe, die Antikörper, insbesondere anti-TGF-β-Antikörper, anti-TGF-α-Antikörper und/oder anti-Interleukin-1-Antikörper, Fab-Fragmente, variable Einzelkettenfragmente (scFv) und multivalente Antikörperfragmente (scFv Multimere) umfasst.

9. Verfahren zum Herstellen eines ophthalmologischen Implantats (10), bei welchem ein Grundkörper (12) mit wenigstens einem am Grundkörper (12) immobilisierten Ligand (L), mittels welchem im implantierten Zustand des ophthalmologischen Implantats (10) zumindest ein Fibrogen und/oder Zytokin gebunden und/oder deaktiviert werden kann, hergestellt wird, wobei auf einem Oberflächenbereich des Grundkörpers (12) ein Schichtsystem mit mindestens zwei Schichten erzeugt wird, wobei wenigstens eine der Schichten den immobilisierten Ligand (L) und wenigstens eine andere der Schichten ein Polymer aus der Gruppe Polyethylenimine, Polyamine und Polyallylamine umfasst.

10. Verfahren nach Anspruch 9,
**dadurch gekennzeichnet, dass**
der wenigstens eine Ligand (L) kovalent an ein Polysaccharid (P) gekoppelt wird, wobei zum kovalenten Koppeln vorzugsweise ein Verfahren durchgeführt wird, das zumindest die Schritte
- Bereitstellen des Liganden (L), wobei dieser wenigstens eine Aminogruppe aufweist;
- Bereitstellen des Polysaccharids (P), wobei dieses wenigstens eine Carbonsäuregruppe aufweist;
- zumindest partielles Aktivieren der Carbonsäuregruppe des Polysaccharids (P);
und
- Koppeln der aktivierten Carbonsäuregruppe mit der wenigstens einen Aminogruppe des Liganden (L)
umfasst.

11. Verfahren nach Anspruch 9 oder 10,
**dadurch gekennzeichnet, dass**
der Grundkörper (12) mit einem Wirkstoffabgabesystem versehen wird, welches dazu ausgebildet ist, im implantierten Zustand des ophthalmologischen Implantats (10) Alkaliionen aus der Gruppe Li⁺, K⁺, Rb⁺ und/oder Cs⁺ abzugeben.

12. Verwendung eines Liganden (L), mittels welchem im implantierten Zustand eines ophthalmologischen Implantats (10) zumindest ein Fibrogen und/oder Zytokin zu binden und/oder zu deaktivieren ist, zur Herstellung eines ophthalmologischen Implantats (10) gemäß einem der Ansprüche 1 bis 8.

## Claims

1. Ophthalmological implant (10) having a main body (12) and at least one ligand (L) immobilized on the main body (12), by means of which at least one fibrogen and/or cytokine is to be bound and/or deactivated when the ophthalmological implant (10) has been implanted, wherein at least part of a surface of the main body (12) has been coated with a layer system which comprises the immobilized ligand (L), wherein the layer system comprises at least two layers and wherein at least one of the layers comprises the immobilized ligand (L) and at least one of the other layers comprises a polymer from the group consisting of polyethylenimines, polyamines and polyallylamines.

2. Ophthalmological implant (10) according to Claim 1,
**characterized in that**
the ligand (L) has been embedded in a matrix and/or in that the ligand (L) has been covalently bonded to at least one polymer, in particular to at least one biopolymer.

3. Ophthalmological implant (10) according to Claim 2,
**characterized in that**
the at least one ligand (L) has been covalently coupled to the at least one polymer via a spacer.

4. Ophthalmological implant (10) according to Claim 2 or 3,
**characterized in that**
the at least one polymer is a polysaccharide (P) from the group consisting of cellulose, a cellulose ether with methyl and/or ethyl and/or propyl groups, in particular hydroxypropylmethylcellulose, hydroxyethylmethylcellulose and/or methylcellulose, a glycosaminoglycan, in particular hyaluronic acid, chondroitin sulfate, dermatan sulfate, heparin, heparan sulfate, keratan sulfate, alginic acid, polymannuronic acid, polyguluronic acid, polyglucuronic acid, amylose, amylopectin, callose, chitosan, polygalactomannan, dextran, xanthan gum and/or a mixture and/or a physiologically acceptable salt, in particular alkali metal salt, thereof.

5. Ophthalmological implant (10) according to any of Claims 1 to 4,
**characterized in that**
it comprises a main body (12) having at least one haptic part and at least one optical part (2, 3), wherein the ligand (L) has preferably been arranged at least on the haptic part (2).

6. Ophthalmological implant (10) according to any of Claims 1 to 5,
**characterized in that**
the main body (12) comprises an accommodation region (VI) in which the immobilized ligand (L) has been arranged and/or **in that** the ligand (L) has been covalently bonded to the main body (12).

7. Ophthalmological implant (10) according to any of Claims 1 to 6,
**characterized in that**
the at least one ligand (L) binds and/or deactivates at least one fibrogen and/or cytokine, in particular TGF-β, TNF-α and/or interleukin-1, occurring in the aqueous humour.

8. Ophthalmological implant (10) according to any of Claims 1 to 7,
**characterized in that**
the at least one ligand (L) is selected from a group comprising antibodies, in particular anti-TGF-β antibodies, anti-TGF-α antibodies and/or antiinterleukin-1 antibodies, Fab fragments, singlechain variable fragments (scFv) and multivalent antibody fragments (scFv multimers).

9. Method for producing an ophthalmological implant (10), in which a main body (12) having at least one ligand (L) immobilized on the main body (12), by means of which at least one fibrogen and/or cytokine can be bound and/or deactivated when the ophthalmological implant (10) has been implanted, is produced, wherein a layer system having at least two layers is generated on a surface region of the main body (12), wherein at least one of the layers comprises the immobilized ligand (L) and at least one of the other layers comprises a polymer from the group consisting of polyethylenimines, polyamines and polyallylamines.

10. Method according to Claim 9,
**characterized in that**
the at least one ligand (L) is covalently coupled to a polysaccharide (P), wherein covalent coupling is preferably achieved by carrying out a method comprising at least the steps of
- providing the ligand (L), this having at least one amino group;
- providing the polysaccharide (P), this having at least one carboxylic acid group;
- at least partially activating the carboxylic acid group of the polysaccharide (P); and
- coupling the activated carboxylic acid group with the at least one amino group of the ligand (L).

11. Method according to Claim 9 or 10,
**characterized in that**
the main body (12) is provided with an activesubstance delivery system designed to deliver alkali metal ions from the group consisting of Li⁺, K⁺, Rb⁺ and/or Cs⁺ when the ophthalmological implant (10) has been implanted.

12. Use of a ligand (L), by means of which at least one fibrogen and/or cytokine is to be bound and/or deactivated when an ophthalmological implant (10) has been implanted, for production of an ophthalmological implant (10) according to any of Claims 1 to 8.

## Revendications

1. Implant ophtalmologique (10) présentant un corps de base (12) et au moins un ligand (L) immobilisé sur le corps de base (12), au moyen duquel ligand, dans l'état implanté de l'implant ophtalmologique (10), au moins un fibrogène et/ou une cytokine est à lier et/ou à désactiver, au moins une partie d'une surface du corps de base (12) étant revêtue par un système stratifié, qui comprend le ligand (L) immobilisé, le système stratifié comprenant au moins deux couches et au moins l'une des couches comprenant le ligand (L) immobilisé et au moins une autre des couches comprenant un polymère du groupe des polyéthylène-imines, des polyamines et des polyallylamines.

2. Implant ophtalmologique (10) selon la revendication 1, **caractérisé en ce que** le ligand (L) est incorporé dans une matrice et/ou **en ce que** le ligand (L) est lié par covalence à au moins un polymère, en particulier à au moins un biopolymère.

3. Implant ophtalmologique (10) selon la revendication 2, **caractérisé en ce que** ledit au moins un ligand (L) est couplé par covalence par l'intermédiaire d'un écarteur audit au moins un polymère.

4. Implant ophtalmologique (10) selon la revendication 2 ou 3, **caractérisé en ce que** ledit au moins un polymère est un polysaccharide (P) du groupe formé par la cellulose, un éther de cellulose présentant des groupes méthyle et/ou éthyle et/ou propyle, en particulier l'hydroxypropylméthylcellulose, l'hydroxyéthylméthylcellulose et/ou la méthylcellulose, un glycosaminoglycane, en particulier l'acide hyaluronique, le sulfate de chondroïtine, le dermatane sulfate, l'héparine, le sulfate d'héparane, le sulfate de kératane, l'acide alginique, l'acide polymannuronique, l'acide polyguluronique, l'acide polyglucuronique, l'amylose, l'amylopectine, le callose, le chitosane, le polygalactomannane, le dextrane, le xanthane et/ou un mélange et/ou un sel physiologiquement acceptable, en particulier un sel de métal alcalin, de ceux-ci.

5. Implant ophtalmologique (10) selon l'une quelconque des revendications 1 à 4, **caractérisé en ce que** celui-ci comprend un corps de base (12) présentant au moins une partie haptique et au moins une partie optique (2, 3), le ligand (L) étant de préférence agencé au moins au niveau de la partie haptique (2).

6. Implant ophtalmologique (10) selon l'une quelconque des revendications 1 à 5, **caractérisé en ce que** le corps de base (12) comprend une zone de réception (VI), dans laquelle est agencé le ligand (L) immobilisé et/ou **en ce que** le ligand (L) est lié par covalence au corps de base (12) .

7. Implant ophtalmologique (10) selon l'une quelconque des revendications 1 à 6, **caractérisé en ce que** ledit au moins un ligand (L) lie et/ou désactive au moins un fibrinogène et/ou une cytokine, en particulier le TGF-ß, le TNF-α et/ou l'interleukine-1 se trouvant dans l'humeur aqueuse.

8. Implant ophtalmologique (10) selon l'une quelconque des revendications 1 à 7, **caractérisé en ce que** ledit au moins un ligand (L) est choisi dans un groupe qui comprend des anticorps, en particulier des anticorps anti-TGF-ß, des anticorps anti-TGF-a et/ou des anticorps antiinterleukine-1, des fragments Fab, des fragments variables simple chaîne (scFv) et des fragments d'anticorps multivalents (multimères scFv).

9. Procédé pour la fabrication d'un implant ophtalmologique (10), dans lequel un corps de base (12) présentant au moins un ligand (L) immobilisé sur le corps de base (12), au moyen duquel ligand, dans l'état implanté de l'implant ophtalmologique (10), au moins un fibrogène et/ou une cytokine peut être lié(e) et/ou désactivé(e), est fabriqué, un système stratifié comprenant au moins deux couches étant réalisé sur une zone de surface du corps de base (12), au moins l'une des couches comprenant le ligand (L) immobilisé et au moins une autre des couches comprenant un polymère du groupe des polyéthylène-imines, des polyamines et des polyallylamines.

10. Procédé selon la revendication 9, **caractérisé en ce que** ledit au moins un ligand (L) est couplé par covalence à un polysaccharide (P), où, pour le couplage par covalence, on réalise de préférence un procédé qui comprend au moins les étapes de
- mise à disposition du ligand (L), celui-ci présentant au moins un groupe amino ;
- mise à disposition du polysaccharide (P), celui-ci présentant au moins un groupe acide carboxylique ;
- activation au moins partielle du groupe acide carboxylique du polysaccharide (P) ; et
- couplage du groupe acide carboxylique activé audit au moins un groupe amino du ligand (L).

11. Procédé selon la revendication 9 ou 10, **caractérisé en ce que** le corps de base (12) est pourvu d'un système de libération de principe actif qui est conçu pour libérer, dans l'état implanté de l'implant ophtalmologique (10), des ions de métal alcalin du groupe Li⁺, K⁺, Rb⁺ et/ou Cs⁺.

12. Utilisation d'un ligand (L) au moyen duquel, dans l'état implanté de l'implant ophtalmologique (10), au moins un fibrogène et/ou une cytokine est à lier et/ou à désactiver, pour la fabrication d'un implant ophtalmologique (10) selon l'une quelconque des revendications 1 à 8.
